# EUROPEAN PATENT APPLICATION

(11) **EP 2 532 250 A1**
(43) Date of publication of application: **12.12.2012**
(21) Application number: 10845253.3
(22) Date of filing: 11.11.2010
(51) Int. Cl.: A23L 1/212

(54) **METHOD FOR MANUFACTURING ALOE POWDER**

(30) Priority: 03.02.2010 JP 2010022227
(71) Applicant: Morinaga Milk Industry Co., Ltd., Minato-ku Tokyo 108-8384 (JP)
(72) Inventor: ASANO, Yuzo, Zama-shi Kanagawa 252-8583 (JP); NOMAGUCHI, Kouji, Zama-shi Kanagawa 252-8583 (JP); YAMADA, Muneo, Zama-shi Kanagawa 252-8583 (JP); TANAKA, Miyuki, Zama-shi Kanagawa 252-8583 (JP)
(74) Representative: Dean, John Paul
(86) International application number: PCT/JP2010/070129
(87) International publication number: WO 2011/096122

(57) **Abstract**

A technique for efficiently performing pulverization of a dried aloe gel using an air flow type mill is provided. 1) A dried aloe gel is treated by supercritical extraction, an extract is removed from the dried aloe gel to obtain an extraction residue, and then 2) the extraction residue is pulverized with an air flow type mill to produce an aloe powder.

## Description

### Technical Field

The present invention relates to an aloe powder that uses dried aloe gel as a raw material, and a method for producing the same.

### Background Art

Plants belonging to the family *Liliaceae*, the genus *Aloe* constitute a class of succulent plants, and include Aloe vera (*Aloe barbadensis* Miller), Krantz aloe (*Aloe arborescens* Miller var. *natalensis* Berger), and so forth. The plants of the genus *Aloe* are known to have various efficacies, and widely used as ingredients of foods, drinks and drugs.

Succulent plants such as those of the genus *Aloe* contain a lot of moisture inside the plants. For example, about 98% or more of gel of Aloe vera (mesophyll of Aloe vera) of the genus *Aloe* consists of moisture, and most of the solid content consists of plant fibers, polysaccharides, and so forth.
As a method of processing plants of the genus *Aloe* having such a characteristic, there are known methods of drying the aloe gel and thereby powdering it.

As such methods, for example, there is known a method of crushing the aloe gel, drying the crushed aloe gel, and then pulverizing the crushed and dried aloe gel. There is also known an aloe gel dry powder prepared by such a method as mentioned above, in which particles have an average particle size of 100 µm or smaller, and 70% by weight or more of the particles have a size of the average particle size ± 30 µm (Patent document 1). As another method, there is also known a method of drying Aloe vera gel by performing microwave heating or simultaneous microwave heating and far-infrared heating under diminished pressure, and uniform microwave heating, and pulverizing the dried product (Patent document 2). In this method, as the pulverizing method, methods utilizing a jet mill, frost shattering and so forth are exemplified. There is also known an aloe powder produced by such methods in which the particles are formed to have an average particle size of 100 µm or smaller.

Meanwhile, as pulverizers for pulverizing food materials, there are known high-speed rotation mills, medium stirring mills, air flow type mills, and so forth, as roughly classified. Among these, the air flow type mills are fine grinding mills in which a material is entrapped in air (gas) or steam jetting from a nozzle at high pressure, and pulverization is attained by collision of the particles or particles and an impinging plate, and since they have advantages such as no temperature elevation in the mills and low risk of contamination of pulverization medium, they are especially suitable for pulverization of materials of foods, drinks, drugs, cosmetics and animal feeds.

For example, there is conventionally known use of a jet mill pulverizer for pulverization of egg shells, and it is reported that a fine egg shell powder having an average particle size of 20 µm or smaller was obtained by this method (Patent documents 3 and 4). Moreover, there is also an example of use of a jet mill pulverizer for pulverization of dried green du zhong leaves (Patent document 5).

By the way, the plants belonging to the genus *Aloe* (also referred to simply as "aloe".) consist of the outer wall of the leaf rind covered with the thick cuticular layer and the mesophyll differentiated into the chlorenchyma cells and the cells with the thin cell walls known as parenchyma existing under the leaf rind.
There is known a method for producing an Aloe vera extract by performing supercritical extraction for dried Aloe vera gel (Patent document 6).
It is also known to perform supercritical extraction for the leaf rind of aloe obtained by removing the aloe vera gel (Non-patent document 1). However, no attention has conventionally been paid at all to the extraction residue obtained by performing supercritical extraction for the dried aloe gel.

### Prior art references

### Patent documents

Patent document 1: Japanese Patent Laid-open (KOKAI) No. 11-192054
Patent document 2: Japanese Patent Laid-open (KOHYO) No. 2008-500023
Patent document 3: Japanese Patent Laid-open (KOKAI) No. 2002-101857
Patent document 4: Japanese Patent Laid-open (KOKAI) No. 2009-89678
Patent document 5: Japanese Patent Laid-open (KOKAI) No. 10-75733
Patent document 6: International Patent Publication WO2007/060911

### Non-patent document

Non-patent document 1: Hu Q. et al., Food Chemistry, 2005, 91, pp.85-90

### Summary of Invention

However, the aloe gel dry powder of Patent document 1 does not have a sufficiently small average particle size. The method for producing an aloe powder of Patent document 2 does not solve the problem that the aloe powder has the characteristic odor of aloe. Moreover, in order to obtain an average particle size of 5 µm or smaller in the aloe powder by this method, moisture content must be reduced to 2% or less.
Patent documents 3 to 5 do not disclose any method for producing an aloe powder.
As described above, as the method for producing an aloe powder, methods of drying aloe gel and then pulverizing the dried product as it is have been conventionally common.
However, if it is attempted to produce an aloe powder by using an air flow type mill, there arises a problem that the hardness of the dried aloe gel as the material is low, therefore collisional energy of particles in the material and the particles and an impinging plate is absorbed by the particles, and the collisional energy cannot be efficiently used for the pulverization.

Therefore, it has been difficult to produce an aloe powder having a small and uniform particle size by the conventional methods, even if an air flow type mill is used. Moreover, the conventional aloe powders produced by the conventional production methods do not have sufficient fluidity. Therefore, aloe powders produced by such methods have a problem that, for example, if they are put into the mouth or applied to the skin, persons feel roughness, or particles settle out in drinks etc.

Aloe powders not having sufficient fluidity also have a problem that they easily cause clogging and so forth in apparatuses on the production line, and therefore handling thereof is not easy.

Furthermore, conventional aloe powders produced by the conventional production methods also have a problem that they have the characteristic odor of aloe.

Against such a background, a technique for efficiently pulverizing dried aloe gel by using an air flow type mill has been desired. There have also been desired an aloe powder having fine particle sizes showing a sharp particle size distribution and sufficient fluidity, and a method for producing it. In addition, there have also been desired an aloe powder having less odor characteristic to aloe and having favorable taste, and a method for producing it.

Therefore, an object of the present invention is to provide a technique for efficiently pulverizing dried aloe gel by using an air flow type mill.
Another object of the present invention is to provide an aloe powder having fine particle sizes showing a sharp particle size distribution and having sufficient fluidity, and a method for producing it.
Still another object of the present invention is to provide an aloe powder having favorable taste and a method for producing it.

While the inventors of the present invention conducted various researches about application of various ingredients contained in the plants of the genus Aloe to foods, drinks and so forth, they found that extraction residue obtained by treating dried aloe gel by supercritical extraction had favorable properties suitable for pulverization by an air flow type mill.
The inventors of the present invention further found that if dried aloe gel was treated by supercritical extraction to remove extract before pulverization using an air flow type mill, the pulverization could be efficiently performed, and an aloe powder produced by such a method had favorable taste, and accomplished the present invention.
The inventors of the present invention further found that an aloe powder having fine particle sizes showing a sharp particle size distribution and having sufficient fluidity could be produced by adjusting pulverization air volume in the air flow type mill, and accomplished the present invention.
The present invention is thus as follows.

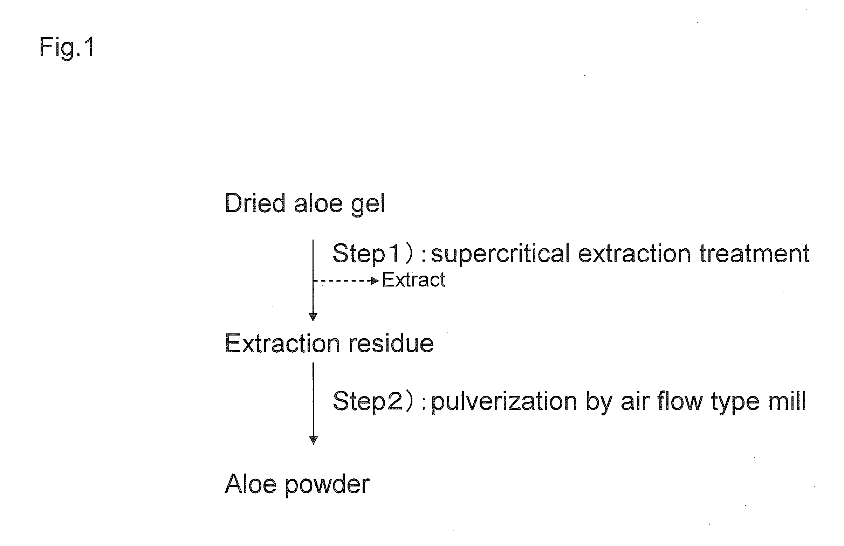

The first invention of the present invention is a method for producing an aloe powder, which comprises the following steps 1) and 2) (henceforth also referred to as the "method for producing an aloe powder of the present inventions") :
1) the step of treating a dried aloe gel by supercritical extraction, and removing an extract from the dried aloe gel to obtain an extraction residue, and
2) the step of pulverizing the extraction residue obtained in the step 1) with an air flow type mill to produce an aloe powder.
The steps of the method for producing an aloe powder of the present invention are shown in Fig. 1.
By using such a method as described above, it becomes possible to efficiently produce an aloe powder by using an air flow type mill. Moreover, it also becomes possible to produce an aloe powder having favorable taste.

In the first invention of the present invention, the air flow type mill is preferably a fluid bed opposed jet air flow type mill.
Since a pulverizer of such type can secure a large solid-gas mixture ratio, it can improve efficiency of comminution, and therefore it contributes to efficient production of aloe powder. Moreover, since a pulverizer of such type utilizes collisional energy of particles in the material to be pulverized, it can eliminate risks of impurity contamination etc. due to abrasion of machine parts, and so forth.

When a fluid bed opposed jet air flow type mill is used, pulverization air volume is preferably 30 m³ or more with respect to 1 kg of the extraction residue.
If the pulverization air volume is within the aforementioned range, an aloe powder having sharp particle size distribution, fine particle size, and sufficient fluidity can be produced.

In the first invention of the present invention, the treatment by supercritical extraction in the step 1) is performed, for example, under the following conditions a) to d).
a) Extraction solvent is carbon dioxide gas.
b) Extraction temperature is 31 to 80°C.
c) Pressure is 7 to 60 MPa.
d) Extraction time is 30 seconds to 7 hours.
If the supercritical extraction is performed under such conditions, 2,4-heptadienal or the like, which is a component as an origin of the grassy smell peculiar to aloe (odor compound), can be sufficiently removed. Moreover, a material (sample) having properties suitable for the air flow type mill can be obtained.

Further, the first invention of the present invention is preferably a method for producing of an aloe powder satisfying the following requirements e) and f).
e) Median size is 5.4 µm or smaller, and 90% particle size is 13.4 µm or smaller.
f) Angle of repose is 56.0 degrees or smaller.

Furthermore, the first invention of the present invention is preferably a method for producing an aloe powder satisfying the following requirement g). g) 2,4-Heptadienal content is 383 mass ppb or lower.

In the first invention of the present invention, the aloe is preferably Aloe vera or Krantz aloe.

The second invention of the present invention is an aloe powder produced by the method for producing an aloe powder of the first invention of the present invention.
In the second invention of the present invention, the aloe powder preferably satisfies the following requirements
e) and f).
e) Median size is 5.4 µm or smaller, and 90% particle size is 13.4 µm or smaller.
f) Angle of repose is 56.0 degrees or smaller.
Such an aloe powder has very smooth texture or feel, and has sufficient fluidity.

In the second invention of the present invention, the aloe powder preferably satisfies the following requirement g) .
g) 2,4-Heptadienal content is 383 mass ppb or lower.

Such an aloe powder gives extremely less and weak odor peculiar to aloe, and has favorable taste.

In the second invention of the present invention, the aloe is preferably Aloe vera or Krantz aloe.

The third invention of the present invention is an aloe powder satisfying the following requirements e) and f).
e) Median size is 5.4 µm or smaller, and 90% particle size is 13.4 µm or smaller.
f) Angle of repose is 56.0 degrees or smaller.

Such an aloe powder has extremely smooth texture or feel, and has sufficient fluidity.

In the third invention of the present invention, the aloe powder preferably satisfies the following requirement g) .
g) 2,4-Heptadienal content is 383 mass ppb or lower.
Such an aloe powder gives extremely less and weak odor peculiar to aloe, and has favorable taste.

As for the aloe powders of the second and third inventions of the present inventions, molar weight of polysaccharides contained in the aloe powders is preferably 100,000 daltons or more, more preferably 400,000 daltons or larger, still more preferably 1 million (1,000,000) daltons or larger.

The effects attainable by the present invention are as follows.
(i) It becomes possible to efficiently produce an aloe powder using an air flow type mill.
(ii) There is provided an aloe powder consisting of fine and highly uniform particles and showing smooth texture and feel.
(iii) There is provided an aloe powder having sufficient fluidity, which thus can be easily handled in the production process.
(iv) There is provided an aloe powder consisting of fine and highly uniform particles, which thus hardly precipitate in a composition (shows high dispersion stability).
(v) There is provided an aloe powder from which bad odor compounds peculiar to aloe are removed.
(vi) The aloe powder of the second invention of the present invention and the aloe powder of the third invention of the present invention (also collectively referred to as "the aloe powder of the present invention") show smooth texture and feel, or favorable taste and flavor, and therefore they are suitable as ingredients of foods, drinks, drugs, cosmetics and animal feeds.
(vii) Since the aloe powder of the present invention can be easily handled in the production process, various uses thereof are expected.

### Brief Description of Drawings

Fig. 1 shows a flowchart of the method for producing an aloe powder of the present invention.
Fig. 2 shows cross-sectional computed tomography (CT) images and three-dimensional computed tomography image of dried aloe gel before subjected to supercritical extraction treatment (photographs).
Fig. 3 shows cross-sectional computed tomography images and three-dimensional computed tomography image of dried aloe gel after subjected to supercritical extraction treatment (photographs).

### Description of Embodiments

Hereafter, preferred embodiments of the present invention will be explained in detail. However, the present invention is not limited by the following preferred embodiments, but can be freely modified within the scope of the present invention.

### <Method for producing aloe powder of the present invention>

### 1) Step of obtaining supercritical extraction residue of dried aloe gel

This step is a step of performing a supercritical extraction treatment for dried aloe gel and removing extract to obtain an extraction residue.

The "dried aloe gel" is a dried product of mesophyll of a plant belonging to the family *Liliaceae,* the genus *Aloe* (in this specification, referred to as "aloe gel"). Examples of the plant belonging to the aforementioned genus *Aloe* include Aloe vera (*Aloe barbadensis* Miller), Krantz aloe (*Aloe arborescens* Miller var. *natalensis* Berger), Aloe ferox (*Aloe ferox* Miller), and so forth. Among these, Aloe vera and Krantz aloe are preferably used.
If an aloe leaf is horizontally sliced, the outer wall of the leaf rind covered with a thick cuticular layer appears. Under the leaf rind, there is the mesophyll differentiated into the chlorenchyma cells and the cells with the thin cell walls known as parenchyma. The parenchyma cells reserve transparent mucilage-like jellies. The fibrovascular bundle with internal bundle sheath cells contains yellow sap having a property of a laxative agent, and exists between two kinds of large cells. That is, aloe has two kinds of major liquid sources, yellow sap (exudate) and clear gel (mucilage). The clear gel (mucilage) is called aloe mesophyll (aloe gel). As described above, a leaf of aloe can be divided into three parts, (A) yellow sap, (B) aloe gel, and (C) leaf skin consisting of leaf rind, tip, base and prickles.
The "dried aloe gel" referred to in the present invention refers to a dried product of (B) the aloe mesophyll (aloe gel).
Of course, the dried aloe gel used in the present invention may contain (A) the yellow sap and (C) the leaf skin in addition to (B) the aloe gel mentioned above. However, when Aloe vera is used as the aloe, neither (A) the yellow sap nor (C) the leaf skin is preferably contained.

The dried aloe gel can be prepared in a conventional manner. For example, it can be prepared through the following steps.

### (i) Step of collecting aloe gel

Leaf skins are peeled from aloe leaves, and the yellow sap is removed by washing.

### (ii) Step of preparing dried aloe gel

The aloe gel collected in (i) is dried to remove moisture. As the method for drying the aloe gel, it can be dried by using a hot air drying machine or freeze drying machine (for example, those produced by Kyowa Vacuum Engineering Co., Ltd.), but the drying method is not particularly limited. If moisture is removed, the aloe gel becomes coarse powdery material.

Further, the dried aloe gel can also be prepared by drying commercially available aloe gel. Further, commercially available dried aloe gel may also be used.

If dried aloe gel is treated by supercritical extraction, an extract is separated from the dried aloe gel. The separated extract is removed.
By such a treatment, the dried aloe gel is divided into the extract and an extraction residue separated from the extract. Among these, the extraction residue is used as a material of the step 2) explained later.

Although the extraction solvent used in this supercritical extraction is not particularly limited, it is preferably a solvent that can extract the components as the origin of the grassy smell peculiar to aloe, for example, butyric acid, 2,4-heptadienal, and 2-ethylhexanol, especially 2,4-heptadienal. For example, it is possible to use carbon dioxide gas, supercritical propane, supercritical ethylene, supercritical 1,1,1,2-tetrafluoroethane, or the like.
When the aloe powder produced by the production method of the present invention is used as an ingredient of foods, drinks, drugs, cosmetics and animal feeds, it is preferable to use carbon dioxide gas as the extraction solvent in view of making a point of safety.

Butyric acid is a colorless liquid having an unpleasant acidic smell like that of rotten butter. Further, 2,4-heptadienal is a colorless liquid giving stimulative green aroma, i.e., grassy smell. 2-Ethylhexanol is a colorless liquid giving rose-like floral aroma, but it may give unpleasant smell depending on quantity.

Extraction temperature can be appropriately chosen according to type of the extraction solvent, and other conditions, and it may be chosen to be, for example, 28 to 120°C as a tentative range. From the aspect of sufficiently extracting the aforementioned odor compounds peculiar to aloe or obtaining properties suitable for the pulverization explained later, the extraction temperature can be chosen to be preferably 31 to 80°C, more preferably 50 to 69°C, still more preferably 50 to 59°C, as a tentative range.

Pressure may be appropriately chosen according to type of the extraction solvent and other conditions, and it may be chosen to be, for example, 5.5 to 60 MPa, as a tentative range. From the aspect of sufficiently extracting the aforementioned odor compounds peculiar to aloe or obtaining properties suitable for the pulverization explained later, the pressure can be chosen to be preferably 7 to 60 MPa, more preferably 15 to 60 MPa, still more preferably 15 to 24 MPa, as a tentative range.

Extraction time can be appropriately chosen according to type of the extraction solvent and other conditions, and it may be chosen to be, for example, 30 seconds to 7 hours, as a tentative range.

As the extraction conditions, for example, the following conditions a) to d) can be mentioned.
a) Extraction solvent is carbon dioxide gas.
b) Extraction temperature is 31 to 80°C.
c) Pressure is 7 to 60 MPa.
d) Extraction time is 30 seconds to 7 hours.

Further, in the supercritical extraction, an entrainer such as ethanol can also be used in order to sufficiently extract the aforementioned odor compounds peculiar to aloe.

### 2) Step of producing aloe powder by pulverizing extraction residue with air flow type mill

This step is a step of pulverizing the extraction residue obtained in the aforementioned step 1) with an air flow type mill.
Examples of the air flow type mill include those of fluid bed opposed jet mill type (counter jet mill, cross jet mill etc.), suction type (jetmizer, micronizer, etc.), jet nozzle type (supersonic jet mill PJM etc.), and collider type (Majac mill, I-type jet mill, etc.).

In this step, among the air flow type mills, a fluid bed opposed jet air flow type mill is particularly preferably used.
In the fluid bed opposed jet air flow type mill, pulverization is attained by jetting compressed air of several atmospheres or more from injection nozzles, accelerating material particles with jet streams jetted from the nozzles, and colliding the accelerated particles at the crossing point of the jet streams. Since a pulverizer of such type can secure a large solid-gas mixture ratio, it can improve efficiency of comminution. Moreover, since a pulverizer of such type utilizes collisional energy of the material particles, it can eliminate risks of impurity contamination etc. due to abrasion of machines, and so forth.
As the fluid bed opposed jet air flow type mill mentioned above, for example, Counter Jet Mill 100AFG produced by Hosokawa Micron Corporation can be used.

The extraction residue obtained in the step 1) mentioned above is continuously supplied to the air flow type mill, and compressed air is blown from nozzles installed in the inside of the pulverizer from the start of the supply. In the present invention, the compressed air blown into the pulverizer is defined as "pulverization air", and volume thereof is defined as "pulverization air volume".

Pressure of the pulverization air is preferably higher than the atmospheric pressure in order to produce a strong flow of the air in the pulverizer, but it can be suitably changed depending on type and size of the pulverizer. When a fluid bed opposed jet air flow type mill is used, the pressure of the pulverization air is preferably 3 MPa or higher, more preferably 4 MPa or higher.

Volume of the pulverization air can be appropriately determined according to type of the pulverizer, chamber volume and chamber geometry of the pulverizer in consideration of desired degree of pulverization. The pulverization air volume is usually 30 to 3000 m³/hour as a tentative range.
Further, rate or speed of supplying the extraction residue into the pulverizer can be appropriately determined according to type of the pulverizer, chamber volume and chamber geometry of the pulverizer like the pulverization air volume. In the pulverizer, concentration of the material in the chamber (material volume/chamber volume of the pulverizer) affects the efficiency of comminution, and preferred concentration in the pulverizer may differ depending on type of the pulverizer. The supplying rate of the extraction residue is usually 1 to 100 kg/hour as a tentative range.

On the other hand, the preferred range of the pulverization air volume relative to the amount of the supplied extraction residue is constant irrespective of size of the pulverizer. In view of realizing extremely sharp particle size distribution and ultrafine particle size, when a fluid bed opposed jet air flow type mill is used, pulverization air volume used for 1 kg of the extraction residue is preferably 30 m³ or more, more preferably 37 m³ or more, still more preferably 50 m³ or more. If the pulverization air volume is in such a range, impact at the time of the collision of the particles becomes strong, and number of times of the collision also increases. Therefore, ultrafine grinding can be attained in such a degree that has not been attained in the conventional pulverization of aloe gel as a material.

Further, the air flow type mill used in the present invention preferably has a control means for controlling the pulverization air volume according to the concentration of the extraction residue in the pulverizer. This control means preferably has a function for automated control of the pulverization air volume according to a control program.
The control means controls apertures of the injection nozzles etc. so that the pulverization air volume for 1 kg of the extraction residue becomes preferably 30 m³ or more, more preferably 37 m³ or more, still more preferably 50 m³ or more.

The aloe powder as the object of the production by the production method of the first invention of the present invention has a median size of preferably 5.5 µm or smaller, more preferably 5.4 µm or smaller, still more preferably 5.0 µm or smaller, further preferably 4.8 µm or smaller. Further, this aloe powder has a 90% particle size of preferably 13.5 µm or smaller, more preferably 13.4 µm or smaller, still more preferably 13.0 µm or smaller, further preferably 12.0 µm or smaller. This aloe powder has an angle of repose of preferably 56.3 degrees or smaller, more preferably 56.0 degrees or smaller, still more preferably 55.0 degrees or smaller, further preferably 50.0 degrees or smaller, particularly preferably 47.4 degrees or smaller.
The methods for measuring these properties will be explained later.

The aloe powder as the object of the production by the production method of the first invention of the present invention preferably satisfies the following requirements
e) and f).
e) Median size is 5.4 µm or smaller, and 90% particle size is 13.4 µm or smaller.
f) Angle of repose is 56.0 degrees or smaller.

Further, the aloe powder as the object of the production by the production method of the first invention of the present invention has a content of 2,4-heptadienal in the aloe powder of preferably 383 mass ppb or lower, more preferably 380 mass ppb or lower, further preferably 350 mass ppb or lower, further preferably 300 mass ppb or lower, further preferably 250 mass ppb or lower, further preferably 233 mass ppb or lower, further preferably 200 mass ppb or lower, further preferably 100 mass ppb or lower, further preferably 50 mass ppb or lower, still further preferably 10 mass ppb or lower.

### <Aloe powder of the present invention>

The aloe powder of the second invention of the present invention is an aloe powder produced by the aforementioned method for producing an aloe powder of the present invention.
The aloe powder of this invention has the following properties.
First, the aloe powder of this invention has a larger density and higher hardness of particles compared with aloe powders produced in the same manner without performing the treatment by supercritical extraction. This is because tissues of the dried aloe gel are consolidated by the treatment of supercritical extraction as shown in the examples mentioned below.

The aloe powder of the second invention of the present invention preferably has a median size of preferably 5.5 µm or smaller, more preferably 5.4 µm or smaller, still more preferably 5.0 µm or smaller, further preferably 4.8 µm or smaller. Further, this aloe powder has a 90% particle size of preferably 13.5 µm or smaller, more preferably 13.4 µm or smaller, still more preferably 13.0 µm or smaller, further preferably 12.0 µm or smaller. This aloe powder has an angle of repose of preferably 56.3 degrees or smaller, more preferably 56.0 degrees or smaller, still more preferably 55.0 degrees or smaller, further preferably 50.0 degrees or smaller, particularly preferably 47.4 degrees or smaller.
The methods for measuring these properties will be explained later.

The aloe powder of the second invention of the present invention preferably satisfies the following requirements e) and f).
e) Median size is 5.4 µm or smaller, and 90% particle size is 13.4 µm or smaller.
f) Angle of repose is 56.0 degrees or smaller.

The aloe powder of the second invention of the present invention has a content of 2,4-heptadienal of preferably 383 mass ppb or lower, further preferably 380 mass ppb or lower, further preferably 350 mass ppb or lower, further preferably 300 mass ppb or lower, further preferably 250 mass ppb or lower, further preferably 233 mass ppb or lower, further preferably 200 mass ppb or lower, further preferably 100 mass ppb or lower, further preferably 50 mass ppb or lower, still further preferably 10 mass ppb or lower.

As for the aloe powder of the second invention of the present invention, the aloe is preferably Aloe vera or Krantz aloe.

The aloe powder of the third invention of the present invention is aloe powder that satisfies the following requirements e) and f).
e) Median size is 5.4 µm or smaller, and 90% particle size is 13.4 µm or smaller.
f) Angle of repose is 56.0 degrees or smaller.
The aloe powder having such properties has smooth texture or feel. Further, handling thereof in processing is also easy.

The aloe powder of the third invention of the present invention has a median size of preferably 5.0 µm or smaller, more preferably 4.8 µm or smaller, a 90% particle size of preferably 12.0 µm or smaller, and an angle of repose of preferably 47.4 degrees or smaller.
By making the aloe powder have the properties within the aforementioned ranges, texture and feel thereof become smoother, and handling thereof in processing also becomes still easier.

The aloe powder of the third invention of the present invention has a content of 2,4-heptadienal of preferably 383 mass ppb or lower, further preferably 380 mass ppb or lower, further preferably 350 mass ppb or lower, further preferably 300 mass ppb or lower, further preferably 250 mass ppb or lower, further preferably 233 mass ppb or lower, further preferably 200 mass ppb or lower, further preferably 100 mass ppb or lower, further preferably 50 mass ppb or lower, still further preferably 10 mass ppb or lower.

In the aloe powders of the second and third inventions of the present inventions, molar weight of polysaccharides contained in the aloe powder is preferably 100,000 daltons or larger, more preferably 400,000 daltons or larger, still more preferably 1 million daltons or larger.

Although the aloe powder of the third invention of the present invention can be produced by the method for producing an aloe powder of the first invention of the present invention, it is not limited to one produced by that method.

### <Methods for measuring properties of aloe powder>

### [1] Median size and 90% particle size

Median size is a particle size at 50% in measured cumulative particle size distribution on volumetric basis, and may also be called 50% particle size or D50, and it may be used as a mean particle size. A smaller median size means that the whole particles have smaller particle sizes.

Further, 90% particle size is a particle size at 90% in cumulative particle size distribution, and may also be called 90% particle size or D90. A smaller difference of the median size and the 90% particle size means more constant particle sizes, i.e., smaller fluctuation of particle sizes.

The median size and 90% particle size referred to in the present invention can be obtained from particle size distribution measured with a particle size measurement system "Mastersizer 2000 Dry System" (produced by Horiba, Ltd.). The refractive index of particles is set at 1.520.
This system is an apparatus utilizing the principle of laser diffraction and scattering method, and with this apparatus, volumetric basis particle size distribution can be obtained by detecting sizes of individual particles from a scattering pattern of laser beam.

### [2] Angle of repose

Angle of repose means an angle between a dip of slope of deposited powder layer and horizontal plane, and it is known to be used as an index indicating fluidity of powders (for example, Powder Technology Handbook, 2nd edition, The Society of Powder Technology, Japan, 1998, page 237). A smaller angle of repose means a higher fluidity, which results in a lower possibility of retention of powders on a production line, and thus easier handling.
Although the measurement methods of the angle of repose is roughly classified into three types, injection method, ejection method, and gradient method, the angle of repose used in the present invention is preferably a value measured by the injection method. In the injection method, powders are flown on a horizontal plane from the above so that the particles are deposited in a conical shape, and angle of slope thereof is measured.
In the present invention, angle of slope of a mountain of fine particles formed by free fall of the aloe powder is automatically measured by image analysis. The angle of repose referred to in the present invention can be measured by using, for example, "Powder Tester PT-S" (produced by Hosokawa Micron Corporation).

### [3] Amount of odor compounds

Measurement of odor compounds can be performed for odor generated when a sample is suspended in water of which temperature is controlled, and odor compounds can be analyzed by a solid phase microextraction gas chromatograph mass spectrometer (GC/MS) or the like.
In the present invention, amount of 2,4-heptadienal can be calculated by numerically evaluating the area thereof in a measured chromatogram. In this case, by creating calibration curves using standard substances, each of odor compounds can be quantified.

Analytical conditions for odor compounds are as follows.

### [Measurement apparatuses]

- GC: Model 6890 produced by Agilent Technologies, Inc.
- MS: Model 5973A produced by Agilent Technologies, Inc.
- Column: INNOWAX (trade name, produced by Agilent Technologies, Inc., internal diameter: 0.25 mm length: 30 m, film thickness: 0.25 µm
- SPME fiber: SUPELCO product

### [Method for separation and concentration of odor compounds]

- Solid phase microextraction method (SPME): 35°C, 30-minute headspace method

### [Measurement conditions]

- Temperature of GC injection point: 265°C
- Gas flow rate: 1.2 ml/minute
- Temperature elevation condition of helium gas oven: 40°C for 2 minutes, 4°C/minute (up to 120 minutes), 6°C/minute (up to 240 minutes), and retention for 10 minutes
- MS measurement mode: scan 2.32 (SCAN/second)

In the present invention, 2,4-heptadienal can be also quantified by HPLC.

The aloe powder of the present invention is preferred as a material of foods, drinks, drugs, cosmetics and animal feeds. That is, the present invention also provides a food, drink, drug, cosmetic or animal feed containing the aloe powder of the present invention.

Hereafter, the present invention will be explained in detail with reference to test examples. In the test examples of the present invention, Aloe vera was used as aloe.

### [Test Example 1]

This test aimed at examining the optimum conditions of the method for producing an aloe powder of the present invention. Further, this test also aimed at comparative study of a sample produced by the production method of the present invention (No. 1) and a sample produced by the production method of Patent document 2 (No. 4).

### (1) Preparation of samples

Leaf skins of 120 tons of Aloe vera were stripped, and the reminder was washed to collect mesophyll parts, which were further dried to prepare 200 kg of dried Aloe vera gel (coarse powder). Then, 100 kg of the prepared dried Aloe vera gel was subjected to supercritical extraction by using a supercritical extraction unit (produced by Uhde High Pressure Technologies GmbH), and the extract was removed to obtain an extraction residue. The treatment conditions were as follows: a) solvent: carbon dioxide gas, b) extraction temperature: 50°C, c) extraction pressure: 15 MPa, and d) extraction time: 60 minutes. On the other hand, 100 kg of the remaining Aloe vera powder was not subjected to the supercritical extraction treatment.
Thus, two kinds of samples that were different only in use or not use of the treatment by supercritical extraction were prepared. Median size of the particles constituting both the samples was 490 µm, and any significant difference of particle size due to use or not use of the supercritical extraction treatment was not observed.

The prepared samples were pulverized under various conditions. For the pulverization, there were used a fluid bed opposed jet air flow type mill "Counter Jet Mill 100AFG" (produced by Hosokawa Micron Corporation), a classifier built-in type high speed mill "trade name: Pulverizer ACM-15" (produced by Hosokawa Micron Corporation), and "Counter Jet Mill 200AFG" (produced by Hosokawa Micron Corporation), which is a large-sized model of the fluid bed opposed jet air flow type mill "Counter Jet Mill 100AFG".
The pressure of the pulverization air used for the pulverization was 0.6 MPa when "Counter Jet Mill 100AFG" was used, or 0.4 MPa when "Counter Jet Mill 200AFG" was used.
When "Counter Jet Mill 100AFG" was used, the pulverization air volume was 22, 30 or 37 m³ for 1 kg of the dried Aloe vera gel continuously supplied in a substantially constant amount per unit time. Further, when "Counter Jet Mill 200AFG" was used, the pulverization air volume was 30 m³ for 1 kg of the dried Aloe vera gel continuously supplied in a substantially constant amount per unit time.

### (2) Test methods

Measurement of particle size distribution was performed for each pulverized sample according to the method described above. For the measurement of particle size, "Mastersizer 2000 Dry System" (produced by Malvern Instruments Ltd.) was used. This apparatus is an apparatus utilizing the principle of laser diffraction and scattering method. The refractive index of particles was set at 1.520, and sizes of the particles were detected from a scattering pattern of the laser beam to obtain volumetric basis particle size distribution.
Angle of repose was measured for a part of the samples. The angle of repose was automatically measured by image analysis as an angle of a mountain formed by free fall of the sample by using Powder Tester PT-S (produced by Hosokawa Micron Corporation) according to the method described above.

### (3) Test results

Use or no use of the supercritical extraction treatment, pulverization air volume, and the measurement results of the median size, 90% particle size and angle of repose of the prepared Aloe vera powders are shown in Table 1.
Among the sample obtained by performing pulverization after performing the supercritical extraction treatment (No. 1) and the sample obtained by performing pulverization without performing the supercritical extraction treatment (No. 4), the sample obtained by performing pulverization after performing the supercritical extraction treatment (No. 1) showed a smaller particle size and higher uniformity of the particles, although the pulverization conditions were the same for both the samples.
Further, there was observed a tendency that a larger pulverization air volume provided a smaller particle size (Nos. 1 to 3).
On the other hand, the sample obtained by using a high speed mill as the pulverizer (No. 5) showed a larger particle size compared with those observed for the samples of Nos. 1 and 2, even when the pulverization was performed after performing the supercritical extraction treatment.

Further, as a fluid bed opposed jet air flow type mill, the large-sized machine "Counter Jet Mill 200AFG" (produced by Hosokawa Micron Corporation) was also used besides "Counter Jet Mill 100AFG" (Nos. 6 and 7). Also in this case, among the sample obtained by performing pulverization after performing the supercritical extraction treatment (No. 6) and the sample obtained by performing pulverization without performing the supercritical extraction treatment (No. 7), the sample obtained by performing pulverization after performing the supercritical extraction treatment (No. 6) showed a smaller particle size and higher uniformity of the particles, although the pulverization conditions were the same for both the samples.
Further, the angles of repose of the samples of Nos. 6 and 7 were measured. The sample of No. 6 showed a smaller angle of repose compared with the sample of No. 7.

The above results revealed as follows.
(i) If the dried aloe gel is treated by supercritical extraction to remove the extract before the pulverization, the pulverization by an air flow type mill can be efficiently performed. This is also effective even when a large amount of extraction residue is treated, and therefore highly practically effective.
(ii) A larger pulverization air volume per unit amount of the extraction residue in the air flow type mill provides a smaller particle size and more improved uniformity of particles.
(iii) If the pulverization air volume is 30 m³ or more per 1 kg of the extraction residue, extremely fine and highly uniform aloe powder can be produced.
(iv) If a dried aloe gel is treated by supercritical extraction to remove the extract, and then the extraction residue is pulverized by an air flow type mill, an aloe powder showing high fluidity can be produced.

**Table 1**

| No. | Supercritical extraction | Pulverizer | Compressed air volume | Aloe vera gel | Compressed air volume/Aloe vera gel | Median size | 90% particle size | Angle of repose |
|---|---|---|---|---|---|---|---|---|
| 1 | Used | A | 37 m³ | 1 kg | 37 m³/kg | 3.5 µm | 8.1 µm | - |
| 2 | Used | A | 30 m³ | 1 kg | 30 m³/kg | 4.7 µm | 11.3 µm | - |
| 3 | Used | A | 22 m³ | 1 kg | 22 m³/kg | 15.6 µm | 54.6 µm | - |
| 4 | Not used | A | 37 m³ | 1 kg | 37 m³/kg | 8.3 µm | 19.9 µm | - |
| 5 | Used | B | - | 1 kg | - | 16.0 µm | 52.0 µm | - |
| 6 | Used | C | 180 m³ | 6 kg | 30 m³/kg | 4.8 µm | 11.8 µm | 47.4° |
| 7 | Not used | C | 180 m³ | 6 kg | 30 m³/kg | 5.5 µm | 13.5 µm | 56.3° |

| | | | | | | | | |
|---|---|---|---|---|---|---|---|---|
| *1 A: Fluid bed opposed jet air flow type mill "Counter Jet Mill 100AFG" (produced by Hosokawa Micron Corporation), B: fixed impact and classifier built-in type high speed mill "Pulverizer ACM-15" (produced by Hosokawa Micron Corporation), C: Fluid bed opposed jet air flow type mill "Counter Jet Mill 200AFG" (produced by Hosokawa Micron Corporation), 2* Pulverization air pressure: 0.6 MPa for Nos. 1 to 4, 0.4 MPa for Nos. 6 and 7 | | | | | | | | |

### [Test Example 2]

This test aimed at examining texture and taste of the Aloe vera powders obtained by the production method of the present invention.

### (1) Preparation of samples

One (1) mass % suspensions of the samples produced in Test Example 1 (Nos. 1 to 7) were prepared.

### (2) Test method

As sensory analyses, texture and odor of the 1 mass % suspensions of the samples were evaluated by 10 panelists.
For the evaluation of texture, the 10 panelists held the 1 mass % suspension of each sample in their mouths, and evaluated "smoothness" and "roughness" thereof on their tongues as sensory analysis. When seven or more panelists sensed "smoothness", it was judged that texture was good (indicated with the symbol ○ in Table 2). Further, when seven or more panelists sensed "roughness", it was judged that texture was bad (indicated with the symbol × in Table 2).
For the evaluation of odor, the 10 panelists held the 1 mass % suspension of each sample in their mouths, and evaluated odor passing through their noses as sensory analysis. When seven or more panelists sensed "freshness", it was judged that odor was good (O), and when seven or more panelists sensed "grassy smell", it was judged that odor was bad (×).

### (3) Test results

For the evaluation of texture, many of the panelists felt "smoothness" for the samples for which pulverization was performed with a pulverization air volume of 30 m³/kg or more after the supercritical extraction was performed (Nos. 1, 2, 6), and texture thereof was judged to be good. On the other hand, relatively many of the panelists felt "roughness" for the samples for which pulverization was performed without performing the supercritical extraction (Nos. 4, 7). Further, for the sample for which pulverization was performed after the supercritical extraction was performed, but with a pulverization air volume as small as 22 m³/kg (No. 3), relatively many of the panelists felt "roughness". Furthermore, also for the sample for which pulverization was performed with the fixed impact classifier built-in type high speed mill after the supercritical extraction was performed (No. 5), relatively many of the panelists felt "roughness".
Furthermore, for the evaluation of odor, many of the panelists felt "freshness" for all the samples for which pulverization was performed after the supercritical extraction was performed, and odor thereof was judged to be good (Nos. 1 to 3, 5, 6). On the other hand, relatively many of the panelists felt "grassy smell" for the samples for which pulverization was performed without performing the supercritical extraction (Nos. 4, 7).

The above results revealed as follows.
(i) By treating a dried aloe gel with supercritical extraction to remove extract before the pulverization, the odor peculiar to aloe can be reduced.
(ii) By 1) treating a dried aloe gel with supercritical extraction to remove extract before the pulverization, and 2) pulverizing the extraction residue obtained in 1) with a fluid bed opposed jet air flow type mill using pulverization air of 30 m³ or more for 1 kg of the extraction residue, an extremely favorable aloe powder showing superior texture and superior taste can be produced.

**Table 2**

| No. | Texture | Odor |
|---|---|---|
| 1 | ○ | ○ |
| 2 | ○ | ○ |
| 3 | × | ○ |
| 4 | × | × |
| 5 | × | ○ |
| 6 | ○ | ○ |
| 7 | × | × |

### [Test Example 3]

This test aimed at quantifying the odor compounds of the Aloe vera powder obtained by the production method of the present invention. As the samples, there were used the sample No. 1 of Test Example 1 obtained with the production method of the present invention and the sample No. 4 of Test Example 1 obtained by the same production method as that of Test Example 1 except that the supercritical extraction was not performed.
Further, this test also aimed at comparative study of the sample produced by the production method of the present invention (No. 1) and the sample produced by the production method of Patent document 2 (No. 4).

### (1) Preparation of samples

One (1) g each of the Aloe vera powders of Nos. 1 and 4 produced in Test Example 1 was taken, and added to 199 g of water, and the mixture was stirred for 30 minutes to swell the Aloe vera powder. To the swelled Aloe vera powder, 20 g of sodium chloride (produced by Wako Pure Chemical Industries Co., Ltd.) was added, and they were mixed. Finally, 11 g of the mixture was taken into vial tubes as test samples.

### (2) Measurement methods

2,4-Heptadienal as an odor compound was measured under the following conditions.

### [Measurement apparatuses]

- GC: Model 6890 produced by Agilent Technologies, Inc.
- MS: Model 5973A produced by Agilent Technologies, Inc.
- Column: INNOWAX (trade name, produced by Agilent Technologies, Inc., internal diameter: 0.25 mm length: 30 m, film thickness: 0.25 µm
- SPME fiber: SUPELCO product

### [Method for separation and concentration of odor compounds]

- Solid phase microextraction method (SPME): 35°C, 30-minute headspace method

### [Measurement conditions]

- Temperature of GC injection point: 265°C
- Gas flow rate: 1.2 ml/minute
- Temperature elevation condition of helium gas oven: 40°C for 2 minutes, 4°C/minute (up to 120 minutes), 6°C/minute (up to 240 minutes), and retention for 10 minutes
- MS measurement mode: scan 2.32 (SCAN/second)

### (3) Results

The measurement results are shown in Table 3.
2,4-Heptadienal content of the sample No. 1 obtained with supercritical extraction was 233 ppb. On the other hand, 2,4-heptadienal content of the sample No. 4 obtained without supercritical extraction was as high as 384 ppb.
From these results, it was revealed that 2,4-heptadienal was decreased by supercritical extraction.
Further, the odor compound-decreasing tendency obtained by supercritical extraction was also confirmed for butyric acid and 2-ethylhexanol, in addition to 2,4-heptadienal.

**Table 3**

| Odor compound | No. 1 (supercritical extraction was used) | No. 4 (supercritical extraction was not used) |
|---|---|---|
| 2,4-Heptadienal | 233 ppb | 384 ppb |

### [Test Example 4]

This test aimed at showing that decrease of 2,4-heptadienal concentration observed in Test Example 3 greatly improved taste of aloe powder.

### (1) Preparation of samples

2,4-Heptadienal (produced by Tokyo Chemical Industry Co., Ltd.) was dissolved in purified water, and diluted to concentrations of 10 to 1000 ppb, and put into vial bottles as test samples. As a control, a sample of 2,4-heptadienal at a concentration of 1000 ppm was prepared in the same manner, and put into a vial bottle.

### (2) Test method

Odor of the samples of the concentrations of 0 to 1000 ppb prepared in (1) was evaluated by a sensory test by 10 panelists.
For the evaluation of odor, the sample of each concentration in a vial bottle was infiltrated into odor test paper (odor paper), and this was brought close to the nose to evaluate whether odor was sensed.
After the panelists first smelled the sample of 1000 ppm infiltrated into odor test paper as a control of odor, the sensory test was started. The odor was evaluated with two stages, the odor of 2,4-heptadienal was sensed (○), and odor was sensed, but it could not be identified as that of 2,4-heptadienal, or odor was not sensed (×).

### (3) Test results

The results are shown in Table 4. When the concentration was 100 ppb or lower, there was no panelist who sensed the odor of 2,4-heptadienal.
On the other hand, when the concentration was 500 ppb or higher, 90% or more of the panelists sensed the odor of 2,4-heptadienal.
Furthermore, when the concentration was 400 ppb, 70% of the panelists sensed the odor of 2,4-heptadienal. When the concentration was 300 ppb, 60% of the panelists sensed the odor of 2,4-heptadienal.
When the concentration was 200 ppb, 30% of the panelists sensed the odor of 2,4-heptadienal.

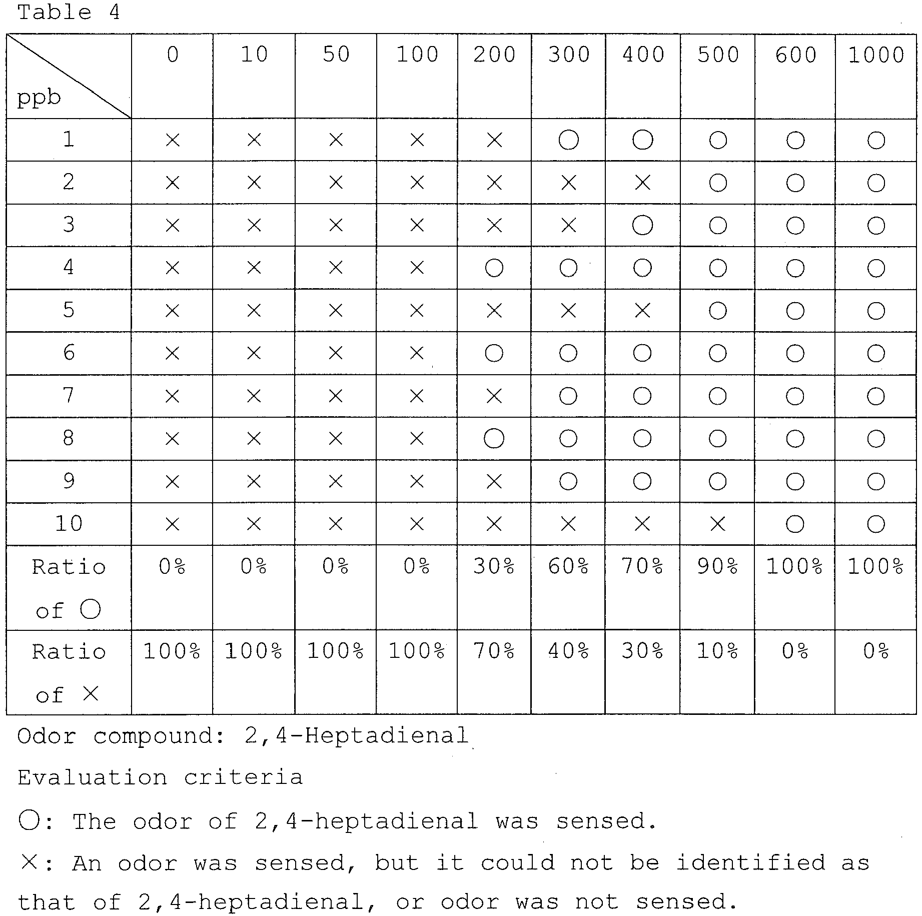

Therefore, the results of the sensory evaluation test revealed that the border of sensing 2,4-heptadienal as an odor or not existed at 300 to 400 mass ppb, especially 380 to 390 mass ppb. In other words, it was suggested that by controlling the 2,4-heptadienal content to be 380 to 390 mass ppb or lower, taste can be controlled on the basis of odor as an index.

As clearly seen from the results mentioned above, the decrease of the concentration of 2,4-heptadienal to 233 mass ppb in the sample obtained with the supercritical extraction treatment (No. 1) compared to the concentration of 384 mass ppb in the sample obtained without the supercritical extraction treatment (No. 4), shown in the results of Test Example 3, means that an aloe powder of which the odor of 2,4-heptadienal was sensed was changed to an aloe powder not existed so far to date of which the odor could not be sensed, i.e., the aloe power produced by the method of the present invention.
In addition, it was revealed that the taste of the aloe powder produced by the method of the present invention was made favorable by decreasing the 2,4-heptadienal content to be lower than at least 384 mass ppb, preferably 383 mass ppb or lower, particularly preferably 233 mass ppb.

### [Test Example 5]

This test aimed at elucidating structural change of the dried aloe gel induced by the supercritical extraction treatment.

### (1) Preparation of samples

There were prepared the dried Aloe vera gel produced in Test Example 1 and the extraction residue obtained by subjecting the dried Aloe vera gel to the supercritical extraction treatment and removing the extract.

### (2) Test method

There were obtained computed tomography images of cross-sections (X-Z cross-section, Y-Z cross-section, X-Y cross-section) of particles having substantially the same maximum particle size contained in the two kinds of samples described in (1) mentioned above, and three-dimensional computed tomography images of the particle, and the structures of the particles in the coarse powders were observed.
As the measurement apparatus, a three-dimensional X-ray computed tomography scanner "TDM1000-IS" (produced by Yamato Scientific Co., Ltd.) was used. The measurement conditions were as follows.

### (i) X-Ray conditions

X-Ray tube voltage: 65 (kV)
Electric current: 0.07 (mA)

### (ii) Scanning parameters

Number of view: 1200
Frame number/view: 12

### (iii) Reconstruction information

Matrix size for X, Y, Z: 512 each
Field of view for X, Y, Z: 0.80 (mm) each

### (3) Test results

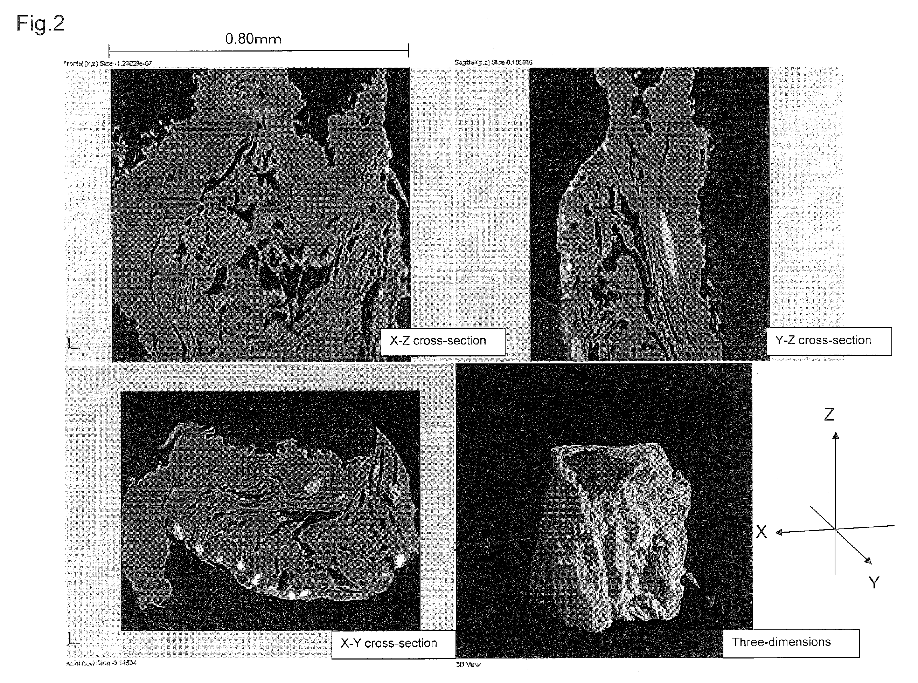

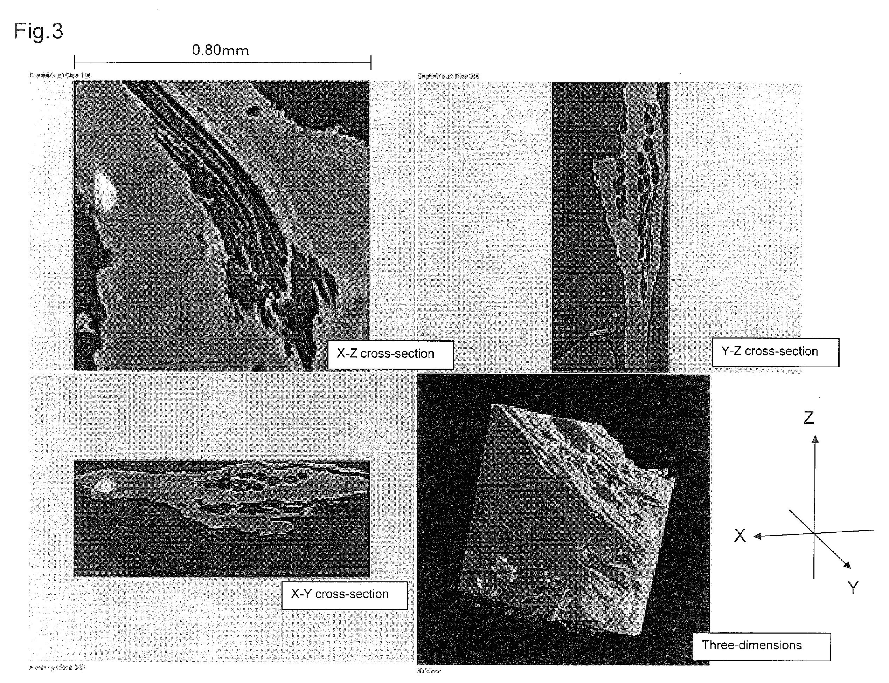

Cross-sectional computed tomography images and a three-dimensional computed tomography image of the dried Aloe vera gel (before the supercritical extraction treatment) are shown in Fig. 2. Further, cross-sectional computed tomography images and a three-dimensional computed tomography image of the extraction residue obtained by the supercritical extraction treatment (after the supercritical extraction treatment) are shown in Fig. 3. In the cross-sectional computed tomography images, the gray parts are Aloe vera mesophyll tissues, and the black parts are voids. Further, the three-dimensional computed tomography images show the appearance of the particles of the dried Aloe vera gel. On the right side of the three-dimensional computed tomography images of Figs. 2 and 3, there are shown the directions of the X-, Y- and Z-axes in the particles of the dried Aloe vera gel. The intersection of the X-, Y- and Z-axes approximately corresponds to the center of gravity of the particle.
As clearly seen from the computed tomography images for the X-Y cross-section and the Y-Z cross-section shown in Figs. 2 and 3, thickness of one side of the Aloe vera gel particle was halved by the supercritical extraction treatment. It is considered that this is because the dried Aloe vera gel was consolidated by the pressure applied by the supercritical extraction treatment.
Further, as seen from comparison of the computed tomography images for the X-Z cross-section shown in Figs. 2 and 3, fine voids were scattered in Fig. 2, whereas the voids aggregated in a large size in Fig. 3.
It is estimated that the large cavity was generated by CO₂ (carbon dioxide) of a supercritical state that passed through the inside of the consolidated dried Aloe vera gel by the supercritical extraction treatment. From the cross-sectional computed tomography images, it is estimated that the Aloe vera powder of the present invention treated by the supercritical extraction came to have a hard and brittle structure, and therefore the efficiency of comminution was improved.

Hereafter, the present invention will be further explained with reference to examples. However, the present invention is not limited to the following examples.

### [Example 1]

Leaf skins of 120 tons of Aloe vera were stripped, and the reminder was washed to collect mesophyll parts, which were further dried to prepare 200 kg of dried Aloe vera gel.
Then, 100 kg of the prepared dried Aloe vera gel was subjected to supercritical extraction by using a supercritical fluid carbon dioxide extraction unit (produced by Uhde High Pressure Technologies GmbH). The extraction conditions were as follows: a) solvent: carbon dioxide gas, b) extraction temperature: 50°C, c) extraction pressure: 15 MPa, and d) extraction time: 60 minutes. The extract was removed by the above treatment to obtain 95 kg of an extraction residue (coarse powder).
Then, 90 kg of the extraction residues was pulverized with a fluid bed opposed jet air flow type mill pulverizer (produced by Hosokawa Micron Corporation). The pulverization was performed with blowing 30 m³ of pulverization air at a pressure of 0.4 MPa per 1 kg of the extraction residue. Supplying time of the extraction residue to the pulverizer was 15 hours, and the pulverization was completed in 15 hours. 85 kg of pulverized Aloe vera powder was obtained.
The produced Aloe vera powder had a median size of 4.8 µm, and a 90% particle size of 11.8 µm. Further, it had an angle of repose of 47.4 degrees, and showed favorable fluidity.
Further, sensory tests by 10 panelists were performed for the produced Aloe vera powder according to the methods described above. As a result, as for texture, 8 panelists out of the 10 panelists sensed "smoothness". As for odor, 9 panelists out of the 10 panelists sensed "freshness". According to the criteria of the aforementioned sensory tests, it was determined that both texture and taste were favorable (○).
As described above, an Aloe vera powder of the present invention showing favorable properties and taste was produced.

### [Example 2]

40 kg of the Aloe vera powder of the present invention produced in Example 1, 40 kg of lactulose (produced by Morinaga Milk Industry Co., Ltd.), 8.5 kg of erythritol (produced by Nikken Chemicals Co., Ltd.), 8 kg of maltitol (produced by Towa Chemical Industry Co., Ltd.), 0.1 kg of stevia (produced by Nippon Paper Chemicals Co., Ltd.), 3 kg of glycerin fatty acid ester (produced by Riken Vitamin Co., Ltd.), and 0.4 kg of yogurt flavor (produced by T. Hasegawa Co., Ltd.) were uniformly mixed, and the mixture was tableted with a rotary tableting machine (produced by Hata Iron Works Co., Ltd.) at a tableting pressure of 2 tons to obtain 195,000 of triangle tablets having a weight of 0.5 g each.

### [Example 3]

30 kg of the Aloe vera powder of the present invention produced in Example 1, 30 kg of lactulose (produced by Morinaga Milk Industry Co., Ltd.), 8.5 kg of xylitol (produced by Towa Chemical Industry Co., Ltd.), 8 kg of maltitol (produced by Towa Chemical Industry Co., Ltd.), 0.1 kg of stevia (produced by Nippon Paper Chemicals Co., Ltd.), 20 kg of dry cell powder of *Bifidobacterium longum,* 3 kg of glycerin fatty acid ester (produced by Riken Vitamin Co., Ltd.), and 0.4 kg of yogurt flavor (produced by T. Hasegawa Co., Ltd.) were uniformly mixed, and the mixture was tableted with a rotary tableting machine (produced by Hata Iron Works Co., Ltd.) at a tableting pressure of 2 tons to obtain 195,000 of triangle tablets having a weight of 0.5 g each.

### [Example 4]

195,000 of triangle tablets having a weight of 0.5 g each were obtained in the same manner as that of Example 3 except that 30 kg of the Aloe vera powder of the present invention produced in Example 1 and 30 kg of lactulose (produced by Morinaga Milk Industry Co., Ltd.) were mixed beforehand, and the mixture was granulated by using a fluid bed spray granulator (produced by Okawara Mfg. Co., Ltd.).

### [Example 5]

1 kg of the Aloe vera powder of the present invention produced in Example 1, 20 kg of squalane, 5 kg of hydrogenated lanolin, 4 kg of cetanol, 4 kg of beeswax, 7 kg of sorbitol, 2 kg of POE(20) sorbitan monooleate, 1.5 kg of glyceryl monostearate, 0.15 kg of methylparaben, 0.1 kg of ethylparaben, a small amount of perfume and purified water were mixed. Further, the mixture was solubilized and emulsified to obtain 100 kg of cream for skin.

### Industrial Applicability

The aloe powder produced by using the method for producing an aloe powder of the present invention can be widely used for foods, drinks, drugs, cosmetics, animal feeds, and so forth.

## Claims

1. A method for producing an aloe powder, which comprises the following steps 1) and 2):
1) the step of treating a dried aloe gel by supercritical extraction, and removing an extract from the dried aloe gel to obtain an extraction residue, and
2) the step of pulverizing the extraction residue obtained in the step 1) with an air flow type mill to produce an aloe powder.

2. The method according to claim 1, wherein the air flow type mill is a fluid bed opposed jet air flow type mill.

3. The method according to claim 2, wherein pulverization air volume is 30 m³ or more for 1 kg of the extraction residue.

4. The method according to any one of claims 1 to 3, wherein the supercritical extraction treatment in the step 1) is performed under the following conditions a) to d) :
a) extraction solvent is carbon dioxide gas,
b) extraction temperature is 31 to 80°C,
c) pressure is 7 to 60 MPa, and
d) extraction time is 30 seconds to 7 hours.

5. The method according to any one of claims 1 to 4, wherein the aloe powder satisfies the following requirements e) and f):
e) median size is 5.4 µm or smaller, and 90% particle size is 13.4 µm or smaller, and
f) angle of repose is 56.0 degrees or smaller.

6. The method according to any one of claims 1 to 5, wherein the aloe powder satisfies the following requirement g):
g) 2,4-Heptadienal content is 383 mass ppb or lower.

7. The method according to any one of claims 1 to 6, wherein the aloe is Aloe vera or Krantz aloe.

8. An aloe powder produced by the method according to any one of claims 1 to 7.

9. The aloe powder according to claim 8, wherein the aloe is Aloe vera or Krantz aloe.
